(19) Europäisches Patentamt
European Patent Office
Office européen des brevets

(11) Publication number: **0 370 165 B1**

(12) **EUROPEAN PATENT SPECIFICATION**

(45) Date of publication of patent specification: **05.01.94**

(51) Int. Cl.[5]: **C07K 7/36**, A61K 37/02

(21) Application number: **89112271.5**

(22) Date of filing: **05.07.89**

(54) **Novel calcitonin derivative and salt thereof.**

(30) Priority: **24.11.88 JP 294761/88**

(43) Date of publication of application:
**30.05.90 Bulletin 90/22**

(45) Publication of the grant of the patent:
**05.01.94 Bulletin 94/01**

(84) Designated Contracting States:
**CH DE ES FR GB IT LI SE**

(56) References cited:
**EP-A- 0 298 474**
**GB-A- 2 184 729**
**US-A- 4 639 510**

(73) Proprietor: **MITSUBISHI PETROCHEMICAL CO., LTD.**
**5-2, Marunouchi 2-chome**
**Chiyoda-ku Tokyo(JP)**

(72) Inventor: **Hane, Motomu c/o Central Research Lab.**
**Mitsubishi Petrochemical Co. Ltd.**
**8-3-1, Chuo**
**Ami-machi Inashiki-gun Ibaraki-ken(JP)**
Inventor: **Hirose, Sachio Mitsubishi Petrochemical Co. Ltd.**
**5-2, Marunouchi 2-chome**
**Chiyoda-ku Tokyo(JP)**
Inventor: **Inagawa, Masanobu c/o Central Research Lab.**
**Mitsubishi Petrochemical Co. Ltd.**
**8-3-1, Chuo**
**Ami-machi Inashiki-gun Ibaraki-ken(JP)**

(74) Representative: **Hansen, Bernd, Dr.rer.nat. et al**
**Hoffmann, Eitle & Partner**
**Patentanwälte**
**Postfach 81 04 20**
**D-81904 München (DE)**

EP 0 370 165 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relates to a novel calcitonin derivative and a salt thereof having the action of lowering the serum $Ca^{2+}$ level and being useful as therapeutic for osteoporosis, bone Paget's disease, digestive hypercalcemia, etc.

Calcitonin is a peptide hormone having a function of lowering calcium in serum and comprising 32 amino acids, and those derived from salmon, eel, chicken, human being, porcine, bovine, sheep and rat have been known. Since these natural type calcitonins have a disulfide bond in the molecule, stability thereof is low in a solution whereby as the therapeutics for digestive hypercalcemia, osteoporosis or bone Paget's disease, synthesized calcitonin derivatives in which the disulfide bond is replaced with an ethylene bond have mainly been used (Japanese Patent Publication No. 41677/1978).

However, since this method must incorporate aminosuberinic acid into a peptide chain, operation is complicated so that an appearance of a derivative which can be obtained with an easy means and retained its biological activity has been demanded.

EP-A-298474 discloses a calcitonin derivative and its salt, comprising an amino acid residue at the 1-position of calcitonin which is a cyclic amine acid residue represented by the formula:

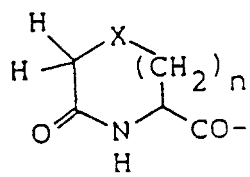

(I)

wherein X represents a sulfur atom or a methylene group; and n represents an integer of 0 or 1, and an amino acid residue at the 7-position which is a cysteine residue which may be substituted with an appropriate protective group.

Said reference is a prior art document in the sense of Art. 54(3) and (4) EPC.

Recently, the present inventors have found that by making an amino acid residue at the 1-position of calcitonin to a specific cyclic amino acid, biological activity and stability of the calcitonin can be improved and synthesis thereof due to solid phase method in peptide chemistry can be realized and have already filed as Japanese Patent Application No. 252592/1987 (which corresponds to European Patent Application No. 88 110 864.1 filed on July 7, 1988).

The present inventors have further studied intensively, and as the results, they have found that high activity and high stability can be admitted in a derivative wherein serine at the 3-position of a chicken calcitonin derivative having the above cyclic amino acid is replaced with threonine to accomplish the present invention.

Also, they have found that by using, as a protective group for the 7-position cystein, an acetamidomethyl group which is a protective group not decomposed under the conditions of HF decomposition after synthesis of a protective peptide, synthesis of a calcitonin derivative can be simplified.

Accordingly, they have synthesized a calcitonin derivative having pyroglutamic acid at the 1-position, threonine at the 3-position and an acetamidomethyl group at the side chain protective group of the 7-position, and have found that this derivative can be more easily synthesized than the already known calcitonin derivatives and has the same or higher activity and stability than the known calcitonin derivatives, to accomplish the present invention.

The abbreviated names, the abbreviated symbols to be used in the present specification have the following meanings.

1. About amino acids:

Ala: alanine, Arg: arginine, Asn: asparagine, Asp: aspartic acid, Cys: cysteine, Gln: glutamine, Glu: glutamic acid, Gly: glycine, His: histidine, Leu: leucine, Lys: lysine, Pro: proline, Ser: serine, Thr: threonine, Tyr: tyrosine, Val: valine, Oct: 3-oxo-5-carboxyperhydro-1,4-thiazine, pGlu: pyroglutamic acid.

In some cases, the respective symbols may show the corresponding amino acid residues.

2. About protective groups:

Boc: t-butyloxycarbonyl, Fmoc: 9-fluorenylmethyloxycarbonyl, $Bu^t$: t-butyl, Bzl: benzyl, $Cl_2$•Bzl: 2,6-dichlorobenzyl, Z: benzyloxycarbonyl, Cl•Z: 2-chlorobenzyloxycarbonyl, Npys: 3-nitropyridinesulphenyl, OBzl: benzyl ester, $OBu^t$: t-butyl ester, OcHex: cyclohexyl ester, Tos: tosyl, Br•Z: 2-bromobenzyloxycarbonyl, $NO_2$: nitro group, Mtr: 4-methoxy-2,3,6-trimethylbenzenesulfonyl, M•Bzl: 4-methoxybenzyl, $4CH_3$•Bzl: 4-methylbenzyl, Trt: trityl, $SBu^t$:t-butylmercapto, Acm: acetamidomethyl.

3. About reagents:

DCC: dicyclohexylcarbodiimide, HOBt: 1-hydroxybenzotriazole, DCM: dichloromethane, DMF: dimethylformamide, DMSO: dimethyl sulfoxide, MeOH: methanol, TEA: triethylamine, TFA: trifluoroacetic acid, HF: hydrofluoric acid, HCl: hydrogen chloride or hydrochloric acid, $CH_3CN$: acetonitrile, Tris HCl: tris-(hydroxymethyl)aminomethane hydrochloride.

4. Others:

CCT: chicken calcitonin,

$\overset{1,7}{\text{Asu}}$-CCT:

derivative wherein cystine residue

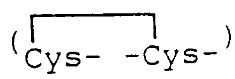

of CCT was replaced with α-aminosuberinic acid residue (Asu:

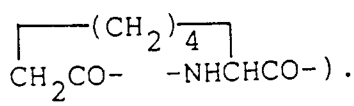

The present invention concerns a novel calcitonin derivative and its salt, represented by the formula:

```
                       Acm
                        |
pGlu-Ala-Thr-Leu-Ser-Thr-Cys-Val-Leu-Gly-Lys-Leu-Ser-Gln-
  Glu-Leu-His-Lys-Leu-Gln-Thr-Tyr-Pro-Arg-Thr-Asp-Val-Gly-
  Ala-Gly-Thr-Pro-NH2
```

wherein Ala represents alanine, Thr: threonine, Leu: leucine, Ser: serine, Cys: cysteine, Val: valine, Gly: glycine, Lys: lysine, Gln: glutamine, His: histidine, Tyr: tyrosine, Pro: proline, Arg: arginine, Asp: aspartic acid, pGlu: pyroglutamic acid, Acm: acetamidomethyl
and Glu: glutamic acid.

The cysteine residue which is the amino acid residue at the 7-position is protected with an acetamidomethyl group.

The calcitonin derivative of the present invention may be in the form of a salt with a mineral acid such as hydrochloric acid, sulfuric acid, phosphoric acid, etc. or an organic acid such as acetic acid, citric acid, etc. , or it may be also in the form of a metal salt such as of sodium, potassium, calcium, etc., or a salt with ammonia or an organic base such as dicyclohexylamine, pyridine, etc.

The calcitonin derivative of the present invention can be synthesized according to the solid phase method or the liquid phase method conventionally used for synthesis of peptides. This synthesis can be performed according to the methods as described in Handbook of Biochemical Experiments, Vol. 1;

"Methods in Protein Chemistry, part D", written by Haruaki Yajima, Shumpei Sakakibara, edited by Society of Biochemistry of Japan, published by Tokyo Kagaku Dojin (1977); and Nobuo Izumiya et al., "Basis and Experiment of Peptide Synthesis", published by Maruzen K.K. (1985). As the synthetic method of the calcitonin derivative of the present invention, the solid phase method is preferred.

In the following, the case of synthesizing the calcitonin derivative of the present invention according to the solid phase method is to be described.

First, Pro, the C-terminal amino acid of the desired calcitonin derivative, is bound to an insoluble resin. Subsequently, protective amino acids are successively bound from the C-terminal side according to the amino acid sequence of said derivative to obtain a protected peptide resin. As the insoluble resin, any of those known in this field of art can be used. For example, chloromethyl resin, hydroxymethyl resin and benzhydrylamine resin (BHA resin) each of which eliminatable with HF, 4-(oxymethyl)-phenoxymethyl resin and 4-(aminomethyl)phenoxymethyl resin each of which eliminatable with TFA, etc. can be employed, and BHA resin or 4-(aminomethyl)phenoxymethyl resin is particularly preferred, since they can give directly amides through cleavage between said resin and the peptide chain.

The "protected amino acid" is an amino acid with the functional group protected with a protective group by the known method, and various protected amino acids are commercially available. In the case of synthesizing the calcitonin derivative of the present invention, it is preferred to select either one of the protective groups shown below. First, the protective group for $\alpha$-amino group is Boc or Fmoc. The protective group for the hydroxyl group of Ser or Thr is $Bu^t$ or Bzl. The protective group for the hydroxyl group of Tyr is $Cl_2$•Bzl, $Bu^t$ or it may not be protected. The protective group for the $\epsilon$-amino group of Lys is Z, Cl•Z, Boc or Npys. The protective group for the carboxyl group of Glu or Asp is OBzl, $OBu^t$ or OcHex. The protective group for the guanidino group of Arg is Tos, $NO_2$ or Mtr. The protective group for the imidazolyl group of His is Tos or Fmoc.

Each protective group is required to be selected suitably depending on the synthetic conditions of the peptide.

Bonding of the protected amino acid can be effected by conventional condensation methods such as the DCC method, the active ester method, the mixed or symmetric acid anhydride method, the cabonyl-diimidazole method, the DCC-HOBt method, the diphenylphosphorylazide method, etc., but the DCC method, the DCC-HOBt method and the symmetric acid anhydride method are preferred. These condensation reactions are generally carried out in an organic solvent such as DCM, DMF, chloroform, DMSO, benzene, or a mixed solvent thereof. It is preferred to carry out such reaction in DCM, DMF or a solvent mixture thereof. As the eliminating reagent of the protective group of $\alpha$-amino group, TFA/DCM, HCl/dioxane, piperidine/DMF, etc. may be employed, and may be selected depending on the kind of said protective group. The extent of the progress of the condensation reaction in the respective stages of synthesis may be examined according to the method of E. Kaiser et al. [Anal. Biochem., 34, p. 595 (1970)] (the ninhydrin reaction method).

As described above, a protected peptide resin having a desired amino acid sequence is obtained, and a specific example thereof is shown below.

```
         Bzl     Bzl Bzl Acm             Cl·Z    Bzl
         |       |   |   |               |       |
pGlu-Ala-Thr-Leu-Ser-Thr-Cys-Val-Leu-Gly-Lys-Leu-Ser-Gln-

OBzl    Tos Cl·Z        Bzl Br·Z    Tos Bzl OBzl
|       |   |           |   |       |   |   |
Glu-Leu-His-Lys-Leu-Gln-Thr-Tyr-Pro-Arg-Thr-Asp-Val-Gly-

        Bzl
        |
Ala-Gly-Thr-Pro-NH-BHA
```

The desired calcitonin derivative is given by means of eliminating the peptide from the resin, and further treating the peptide with a reagent capable of eliminating protective groups other than mercapto group, for example, HF, TFA, etc. (the final deprotecting reaction).

The peptide thus obtained can be isolated and purified according to the conventional methods such as extraction, recrystallization, various chromatography (gel filtration, ion-exchange, partition, adsorption, reverse phase), electrophoresis, counter-current partition, etc., but the method according to the reverse phase high performance liquid chromatography (reverse phase HPLC) is the most effective.

The present invention is described in more detail by referring to the following Example and Reference Example, but said Example is not limitative of the present invention at all.

Reference Example 1

Synthesis of the peptide [(Oct[1], Thr[3], Cys(Acm)[7]-CCT] represented by:

```
                              Acm
                               |
Oct-Ala-Thr-Leu-Ser-Thr-Cys-Val-Leu-Gly-Lys-Leu-Ser-Gln-
Glu-Leu-His-Lys-Leu-Gln-Thr-Tyr-Pro-Arg-Thr-Asp-Val-Gly-
Ala-Gly-Thr-Pro-NH2                               (I)
```

(1) Synthesis of 3-oxo-5-carboxyperhydro-1,4-thiazine:

A solution of 1.75 g (10 mmole) of L-cysteine hydrochloride monohydrate dissolved in 50 ml of water was adjusted to pH 8.0 with 1M sodium carbonate. Under nitrogen gas stream, 1.85 g (10 mmole) of monoiodoacetamide and 1M sodium carbonate were added alternately while maintaining pH at 8.0. After the reaction was carried out at room temperature for 1 hour, further the reaction was carried out in a sealed tube at 100 °C for 2 hours. To the reaction mixture was added 4N HCl to adjust the pH to 3.25, followed by concentration to dryness. The residue was extracted with hot ethanol, and after cooling, the crystals were collected by filtration and recrystallized from hot ethanol. Yield: 560 mg (34.8 %), melting point: 182 to 184 °C, Fab mass analysis $[M + H]^+ = 162$.

(2) Introduction of proline into BHA resin:

An amount of 2 g of BHA resin (produced by Peptide Kenkyusho, divinylbenzene 2 %, 100 to 200 mesh, amino group equivalent: 0.61 meq/g) was placed in a reaction vessel for peptide solid phase synthesis (produced by Dupont Co.), successively treated with each 30 ml of the solvents shown below, and filtered after each treatment.

DCM (three times, each 2 min)
MeOH (three times, each 1 min)
DCM (three times, each 2 min)
10 % TEA/DCM solution (5 min, 10 min, each once)

Subsequently, the BHA resin was stirred together with 0.32 g (1.5 mmole) of Boc-Pro dissolved in 15 ml of DCM for 2 minutes. A solution of 0.31 g (1.5 mmole) of DCC in 15 ml DCM was added, and the mixture was stirred for 120 minutes. The reaction mixture was stirred, and the Boc-Pro-resin was washed with each 30 ml of the following solvents and filtered.

DCM (three times, each 2 min)
MeOH (three times, each 2 min)
DCM (three times, each 2 min)

Further, a solution of 1.34 g (12.2 mmole) of 1-acetylimidazole in 30 ml of DCM was added to the Boc-Pro-resin and acetylation was effected over 12 hours. By this reaction, the unreacted amino groups in the BHA resin were modified, to give subsequently the N-terminal end of proline which becomes the reaction initiation point for addition extension of protective amino acids.

(3) Introduction of the 31-position threonine:

The total amount of the Boc-Pro-resin obtained in (2) was washed with DCM (three times), MeOH (three times) and DCM (three times) each for 2 minutes and filtered. To the resin was added 30 ml of a 50 % TFA solution (solvent: DCM) and the mixture was stirred for 5 minutes, followed by filtration. Further, the resin was stirred under a similar TFA solution for 30 minutes to eliminate Boc groups. The resin obtained was successively treated with each 30 ml of the solvents shown below similarly as in (2), and filtered after each treatment.

DCM (three times, each 2 min)
MeOH (three times, each 2 min)
DCM (three times, each 2 min)
10 % TEA/DCM solution (5 min, 10 min, each once)
DCM (three times, each 2 min)

Subsequently, to the Pro-resin was added a solution of 0.46 g (1.5 mmole) of Boc-Thr(Bzl) in 15 ml DCM, and the mixture was stirred for 2 minutes. Next, a solution of 0.31 g of DCC in 15 ml DCM was added, and the mixture was stirred for 240 minutes. After the reaction, the mixture was washed with DCM (three times), MeOH (three times) and DCM (three times), each for 2 minutes, followed by filtration. A very minute amount of this resin was sampled and confirmed that the ninhydrin test was negative. Next, a part of the resin was sampled, and its C-terminal amino acid bound content was measured to be 0.15 mmole/g.

(4) Introduction of each amino acid at the 30- to 1-positions:

Similarly as described in (3), the Boc-Thr(Bzl)-Pro-resin was coupled successively with the protective amino acids corresponding to the respective constituent amino acids from the 30-position to the 1-position of CT repesented by the above formula (I). Table 1 shows the protective amino acids and their amounts used in the respective reaction steps.

The coupling reaction was conducted twice with the same amounts of the protective amino group, and the coupling reaction time was made 120 minutes for the first time and 300 minutes for the second time. Here, Lys was used as Boc-Lys(Cl•Z) by eliminating TBA of Boc-Lys(Cl•Z)TBA (produced by Peptide Kenkyusho). Since Boc-Leu•$H_2O$ and Oct were difficultly soluble in DCM single solvent, Boc-Leu•$H_2O$was used by dissolving in a solvent mixture of DMF and DCM (1 : 4), and Oct in DMF. Further, DMF was used in place of DCM for the washing solvent during coupling of Oct. In introduction of Gln at the 14- and 20-positions, 0.23 g of HOBt and 0.37 g of Boc-Gln were added into the reaction vessel at the same time.

After introduction of the amino acid of the 1-position, the resin peptide was dried under reduced pressure overnight to obtain a dry resin peptide.

Table 1 Protective amino acids used in **Reference Example** and amounts thereof

| Position of amino acid | Protective amino acid | Molecular weight | Amounts used per single coupling (g) | Remarks |
|---|---|---|---|---|
| 30 | Boc-Gly | 175.1 | 0.26 | |
| 29 | Boc-Ala | 189.2 | 0.28 | |
| 28 | Boc-Gly | 175.1 | 0.26 | |
| 27 | Boc-Val | 217.2 | 0.33 | |
| 26 | Boc-Asp(OBzl) | 323.3 | 0.48 | |
| 25 | Boc-Thr(Bzl) | 309.3 | 0.46 | |
| 24 | Boc-Arg(Tos) | 428.5 | 0.64 | |
| 23 | Boc-Pro | 215.0 | 0.32 | |
| 22 | Boc-Tyr(Br·Z) | 494.3 | 0.74 | |
| 21 | Boc-Thr(Bzl) | 309.3 | 0.46 | |
| 20 | Boc-Gln | 246.2 | 0.37 | 0.23 g of HOBt combinedly used |
| 19 | Boc-Leu·$H_2O$ | 249.3 | 0.37 | |

Table 1 (Contd)

| Position of amino acid | Protective amino acid | Molecular weight | Amounts used per single coupling (g) | Remarks |
|---|---|---|---|---|
| 18 | Boc-Lys(Cl·Z)·TBA | 487.9 | 0.73 | |
| 17 | Boc-His(Tos) | 423.0 | 0.63 | |
| 16 | Boc-Leu·$H_2O$ | 249.3 | 0.37 | |
| 15 | Boc-Glu(OBzl) | 337.3 | 0.51 | |
| 14 | Boc-Gln | 246.2 | 0.37 | 0.23 g of HOBt combinedly used |
| 13 | Boc-Ser(Bzl) | 295.3 | 0.44 | |
| 12 | Boc-Leu·$H_2O$ | 249.3 | 0.37 | |
| 11 | Boc-Lys(Cl·Z)·TBA | 487.9 | 0.73 | |
| 10 | Boc-Gly | 175.1 | 0.26 | |
| 9 | Boc-Leu·$H_2O$ | 249.3 | 0.37 | |
| 8 | Boc-Val | 217.2 | 0.33 | |
| 7 | Boc-Cys(Acm) | 292.3 | 0.44 | |
| 6 | Boc-Thr(Bzl) | 309.3 | 0.46 | |
| 5 | Boc-Ser(Bzl) | 295.3 | 0.44 | |
| 4 | Boc-Leu·$H_2O$ | 249.3 | 0.37 | |
| 3 | Boc-Thr(Bzl) | 309.3 | 0.46 | |
| 2 | Boc-Ala | 189.2 | 0.28 | |
| 1 | Oct | 161.2 | 0.24 | |

(5) Decomposition with HF:

A part (50.7 mg) of the dried resin peptide was weighed, placed in a reaction vessel for HF decomposition (made of Teflon®, trade name) and, with addition of 0.5 ml of anisol, left to stand overnight to swell the resin. The above reaction vessel having a stirrer placed therein was mounted on a HF decomposition device (produced by Peptide Kenkyusho), placed in a dry ice-ethanol bath and 5 ml of HF was introduced into the reaction vessel. The mixture was stirred in an ice-bath at 0 °C for 1 hour. Under reduced pressure, HF was gradually evaporated. After 3 hours, the reaction vessel was dismantled, and the deprotected peptide was taken out from the reaction vessel by use of diethyl ether, and washed with diethyl ether. The deprotected peptide was added into 20 ml of 2 M acetic acid to dissolve the deprotected

peptide.

The solution was passed through a Dowex® (trade name) column of 1 x 2 cm ($AcO^-$) and the eluate was lyophilized to obtain 19.2 mg of crude ($Oct^1$, $Thr^3$, $Cys(Acm)^7$)-CCT.

(6) Purification of crude ($Oct^1$, $Thr^3$, $Cys(Acm)^7$)-CCT:

The crude ($Oct^1$, $Thr^3$, $Cys(Acm)^7$)-CCT obtained was dissolved in a 1 M acetic acid and purified by the reverse phase high performance liquid chromatography. Chemcopack ODS-H (trade name, $\phi$: 10 mm x 250 mm) column produced by Chemco Co. was used, with the eluents employed being water (100) - 10 % TFA (1) for the eluent A and water (40) - acetonitrile (60) - 10 % TFA (1) for the eluent B, and the elution was effected under the linear concentration gradient from the eluent A to the eluent B. Here, the numerals within bracckets represent volume ratios. The fraction corresponding to ($Oct^1$, $Thr^3$, $Cys(Acm)^7$)-CCT was collected and lyophilized to obtain 1.75 mg of white powder.

Its amino acid analytical values are shown in Table 2.

Table 2

| Amino acid composition of ($Oct^1$, $Thr^3$, $Cys(Acm)^7$)-CCT | | |
|---|---|---|
| Amino acid | Molar ratio* | Amino acid number |
| Asx** | 1.02 | 1 |
| Thr | 4.21 | 5 |
| Ser | 1.76 | 2 |
| Glx*** | 2.92 | 3 |
| Pro | 1.96 | 2 |
| Gly | 3.02 | 3 |
| Ala | 1.99 | 2 |
| Val | 1.94 | 2 |
| Leu | 5.00 | 5 |
| Tyr | 0.98 | 1 |
| Lys | 1.96 | 2 |
| His | 1.04 | 1 |
| Arg | 0.97 | 1 |
| Cys(CM)**** | 0.38 | 1 |

* claculated by converting Leu to 5.00.
** Asn and Asp
*** Gln and Glu
**** Oct is hydrolyzed and detected as Cys(CM).

The white powder obtained was assayed by high performance liquid chromatography.

Measurement conditions (1):

Column: Chemcopack ODS-H (trade name, $\phi$: 4.6 mm x 150 mm), produced by Chemco Co.;
Flow rate: 1.0 ml/min;
Eluent: eluent A (water : acetonitrile : 10 % TFA = 100 : 0 : 1)
eluent B (water : acetonitrile : 10 % TFA = 40 : 60 : 1)
were used, and eluted by the linear gradient from the eluent A to the eluent B (30 min);
Measurement wavelength: 210 nm.

As the result, a single strong absorption based on a peptide was recognized at a retention time of 30.0 min, which was (Oct$^1$, Thr$^3$, Cys(Acm)$^7$)-CCT of the present invention.

The result of Fab Mass analysis of the present peptide is shown below:

| | |
|---|---|
| Observed value ($[M ÷ H]^+$) | 3497.2 |
| Theoretical value ($[M + H]^+$) | 3497.8 |

Example 1

Synthesis of:

```
                         Acm
                          |
pGlu-Ala-Thr-Leu-Ser-Thr-Cys-Val-Leu-Gly-Lys-Leu-Ser-Gln-
Glu-Leu-His-Lys-Leu-Gln-Thr-Tyr-Pro-Arg-Thr-Asp-Val-Gly-
Ala-Gly-Thr-Pro-NH2
```

[pGlu$^1$, Thr$^3$, Cys(Acm)$^7$]-CCT

In introduction of the amino acid at the 1-position, solid phase synthesis was carried out under the same conditions as in Reference Example 1 except for using 0.19 g of pGlu in place of Oct, a protected peptide resin was obtained. A part of the protected peptide resin was subjected to HF decomposition in the same manner as in Reference Example 1. The crude peptide obtained was purified in the same manner as in Reference Example 1 - (6), 1.79 mg of (pGlu$^1$, Thr$^3$, Cys(Acm)$^7$)-CCT was obtained.

Test example 1 Biological activity test

Biological activities of the calcitonin derivatives of the present invention were assayed according to the method as described in Japanese Provisional Patent Publication No. 123500/1985 and compared with those of Oct$^1$, Thr$^3$, Cys(Acm)$^7$)-CCT,

$Asu^{1,7}$ CCT

CCT and CCT.

The results are shown in Table 3.

Table 3

| Name of peptide | Activity (MRC U/mg) |
|---|---|
| $(Oct^1, Thr^3, Cys(Acm)^7)$-CCT | 7800 |
| $(pGlu^1, Thr^3, Cys(Acm)^7)$-CCT | 7500 |
| $Asu^{1,7}$ CCT | 4700[1)] |
| CCT | 4500[2)] |

1) Y. Sako, M. Shibata, et al., Peptide Chemistry 1986, 169 (1985)

2) M. Homma, M. Watanabe, et al., J. Biochem., 100, 459 (1986)

Test example 2 Stability test

The stability test of the present product was performed as described below.

The calcitonin derivative was dissolved in 0.1 M sodium citrate buffer (pH 6.0) to a concentration of 10 µg/ml. One ml of this solution was left to stand in a sealed tube at 80 °C for 24 hours. By analysis according to the reverse phase HPLC by use of the measurement conditions (1), the eluted peak area A of the remaining calcitonin derivative was determined. Similarly, the eluted peak area B of the calcitonin derivative in the sample solution not subjected to the heat treatment was determined, and the ratio of A to B (%) was defined as the index. Similarly, the results of the stability test of $Oct^1$, $Thr^3$, $Cys(Acm)^7$)-CCT, CCT and

$$Asu^{1,7}\ CCT$$

CCT are summarized in Table 4.

Table 4 Stability test

| Name of peptide | Stability (%) |
|---|---|
| $(Oct^1, Thr^3, Cys(Acm)^7)$-CCT | 82 |
| $(pGlu^1, Thr^3, Cys(Acm)^7)$-CCT | 90 |
| $Asu^{1,7}$ CCT | 88 |
| CCT | 33 |

The method for preparation of the compounds as described in the foregoing description or in the Example can be altered. For example, as the protected amino acids to be used, a large number of amino acids with different protective groups are commercially available. These commercially available protected amino acids can be used suitably changed, and all of the peptides obtained are equal to the calcitonin derivative of the present invention.

According to the present invention, a calcitonin derivative having a high activity and a high stability can be supplied stably and at low cost. Also, the calcitonin derivative of the present invention can be used as the reagent for experiments, further as the diagnostic agents by establishing an assay system by use thereof, and it can be also utilized as the pharmaceutical or animal medicine on the bases of the biological activity.

**Claims**

**Claims for the following Contracting States : CH, DE, FR, GB, IT, LI, SE**

1. A calcitonin derivative and its salt, wherein the calcitonin derivative is represented by the formula:

```
                         Acm
                          |
pGlu-Ala-Thr-Leu-Ser-Thr-Cys-Val-Leu-Gly-Lys-Leu-Ser-Gln-
 Glu-Leu-His-Lys-Leu-Gln-Thr-Tyr-Pro-Arg-Thr-Asp-Val-Gly-
 Ala-Gly-Thr-Pro-NH2
```

wherein Ala represents alanine, Thr: threonine, Leu: leucine, Ser: serine, Cys: cysteine, Val: valine, Gly: glycine, Lys: lysine, Gln: glutamine, His: histidine, Tyr: tyrosine, Pro: proline, Arg: arginine, Asp: aspartic acid, pGlu: pyroglutamic acid, Acm: acetamidomethyl and Glu: glutamic acid.

2. A calcitonin derivative and its salt according to claim 1, wherein a salt of the calcitonin derivative is a salt of a mineral acid, an organic acid, a metal, ammonia or an organic base.

3. A calcitonin derivative and its salt according to claim 2, wherein a salt of the calcitonin derivative is a salt of hydrochloric acid, sulfuric acid, phosphoric acid, acetic acid, citric acid, sodium, potassium, calcium, ammonia, dicyclohexylamine or pyridine.

4. A method for preparing a calcitonin derivative as claimed in any of the preceding claims by carrying out either the solid phase or the liquid phase method of peptide synthesis.

5. A pharmaceutical composition comprising a calcitonin derivative according to any of claims 1-3 or a salt thereof and a pharmacologically acceptable carrier.

6. A calcitonin derivative according to any of claims 1-3 for use as a medicament.

7. A calcitonin derivative according to any of claims 1-3 for use in lowering the serum $Ca^{2+}$ level in an animal or a human.

8. Use of calcitonin derivative according to any of claims 1-3 for making a medicament effective in lowering serum $Ca^{2+}$ levels or treating osteoporosis, bone Paget's disease or digestive hypercalcemia.

**Claims for the following Contracting State : ES**

1. A method for preparing a calcitonin derivative represented by the formula:

```
                         Acm
                         |
pGlu-Ala-Thr-Leu-Ser-Thr-Cys-Val-Leu-Gly-Lys-Leu-Ser-Gln-
 Glu-Leu-His-Lys-Leu-Gln-Thr-Tyr-Pro-Arg-Thr-Asp-Val-Gly-
 Ala-Gly-Thr-Pro-NH2
```

wherein Ala represents alanine, Thr: threonine, Leu: leucine, Ser: serine, Cys: cysteine, Val: valine, Gly: glycine, Lys: lysine, Gln: glutamine, His: histidine, Tyr: tyrosine, Pro: proline, Arg: arginine, Asp: aspartic acid, pGlu: pyroglutamic acid, Acm: acetamidomethyl
and Glu: glutamic acid, or its salt
by carrying out either the solid phase or the liquid phase method of peptide synthesis.

2. The method according to claim 1 wherein as salt of the calcitonin derivative a salt of a mineral acid, an organic acid, a metal, ammonia or an organic base is prepared.

3. The method according to claim 2 wherein as salt of the calcitonin derivative a salt of hydrochloric acid, sulfuric acid, phosphoric acid, acetic acid, citric acid, sodium, potassium, calcium, ammonia, dicyclohexylamine or pyridine is prepared.

4. A method for preparing a pharmaceutical composition by formulating a calcitonin derivative according to any of claims 1-3 or a salt thereof with a pharmacologically acceptable carrier.

5. Use of a calcitonin dervative and its salt, wherein the calcitonin derivative is represented by the formula:

```
                         Acm
                         |
pGlu-Ala-Thr-Leu-Ser-Thr-Cys-Val-Leu-Gly-Lys-Leu-Ser-Gln-
 Glu-Leu-His-Lys-Leu-Gln-Thr-Tyr-Pro-Arg-Thr-Asp-Val-Gly-
 Ala-Gly-Thr-Pro-NH2
```

wherein Ala represents alanine, Thr: threonine, Leu: leucine, Ser: serine, Cys: cysteine, Val: valine, Gly: glycine, Lys: lysine, Gln: glutamine, His: histidine, Tyr: tyrosine, Pro: proline, Arg: arginine, Asp: aspartic acid, pGlu: pyroglutamic acid, Acm: acetamidomethyl
and Glu: glutamic acid
for making a medicament effective in lowering serum $Ca^{2+}$ levels or treating osteoporosis, bone Paget's disease or digestive hypercalcemia.

6. The use according to claim 5 wherein a salt of the calcitonin derivative is a salt of a mineral acid, an organic acid, a metal, ammonia or an organic base.

**7.** The use according to claim 6 wherein a salt of the calcitonin derivative is a salt of hydrochloric acid, sulfuric acid, phosphoric acid, acetic acid, citric acid, sodium, potassium, calcium, ammonia, dicyclohexylamine or pyridine.

**8.** Use of a calcitonin derivative or its salt as defined in any of claims 1-3 for preparing a medicament.

**Patentansprüche**
**Patentansprüche für folgende Vertragsstaaten : CH, DE, FR, GB, IT, LI, SE**

**1.** Calcitoninderivat und dessen Salz, wobei das Calcitoninderivat durch die Formel:

```
                              Acm
                               |
pGlu-Ala-Thr-Leu-Ser-Thr-Cys-Val-Leu-Gly-Lys-Leu-Ser-Gln-
 Glu-Leu-His-Lys-Leu-Gln-Thr-Tyr-Pro-Arg-Thr-Asp-Val-Gly-
 Ala-Gly-Thr-Pro-NH2
```

dargestellt ist, wobei Ala: Alanin, Thr: Threonin, Leu: Leucin, Ser: Serin, Cys: Cystein, Val: Valin, Gly: Glycin, Lys: Lysin, Gln: Glutamin, His: Histidin, Tyr: Tyrosin, Pro: Prolin, Arg: Arginin, Asp: Asparaginsäure, pGlu: Pyroglutaminsäure, Acm: Acetamidomethyl und Glu: Glutaminsäure bedeutet.

**2.** Calcitoninderivat und dessen Salz nach Anspruch 1, wobei das Salz des Calcitoninderivats ein Salz einer Mineralsäure, einer organischen Säure, eines Metalls, von Ammoniak oder einer organischen Base ist.

**3.** Calcitoninderivat und dessen Salz nach Anspruch 2, wobei das Salz des Calcitoninderivats ein Salz der Salzsäure, Schwefelsäure, Phosphorsäure, Essigsäure, Zitronensäure, von Natrium, Kalium, Calcium, Ammoniak, Dicyclohexylamin oder Pyridin ist.

**4.** Verfahren zum Herstellen eines Calcitoninderivats nach einem der vorhergehenden Ansprüche, indem entweder Feststoffphasen- oder Flüssigphasen-Verfahren der Peptidsynthese durchgeführt wird.

**5.** Pharmazeutische Zusammensetzung, umfassend ein Calcitoninderivat nach einem der Ansprüche 1 bis 3 oder ein Salz davon und einen pharmakologisch geeigneten Träger.

**6.** Calcitoninderivat nach einem der Ansprüche 1 bis 3 für die Verwendung als Arzneimittel.

**7.** Calcitoninderivat nach einem der Ansprüche 1 bis 3 für die Verwendung bei der Verringerung des Serum-$Ca^{2+}$-Spiegels beim Tier oder Menschen.

**8.** Verwendung eines Calcitoninderivats nach einem der Ansprüche 1 bis 3 für die Herstellung eines Arzneimittels, welches wirksam in bezug auf die Verringerung des Serum-$Ca^{2+}$-Spiegels oder die Behandlung von Osteoporose, Paget's Knochenkrankheit oder digestive Hyperkalzämie ist.

**Patentansprüche für folgenden Vertragsstaat : ES**

1. Verfahren zum Herstellen eines Calcitoninderivats, welches durch die Formel:

```
                              Acm
                               |
pGlu-Ala-Thr-Leu-Ser-Thr-Cys-Val-Leu-Gly-Lys-Leu-Ser-Gln-
 Glu-Leu-His-Lys-Leu-Gln-Thr-Tyr-Pro-Arg-Thr-Asp-Val-Gly-
 Ala-Gly-Thr-Pro-NH2
```

dargestellt ist, wobei Ala: Alanin, Thr: Threonin, Leu: Leucin, Ser: Serin, Cys: Cystein, Val: Valin, Gly: Glycin, Lys: Lysin, Gln: Glutamin, His: Histidin, Tyr: Tyrosin, Pro: Prolin, Arg: Arginin, Asp: Asparaginsäure, pGlu: Pyroglutaminsäure, Acm: Acetamidomethyl und Glu: Glutaminsäure bedeutet, oder dessen Salz, indem entweder das Feststoffphasen- oder das Flüssigphasen-Verfahren der Peptidsynthese durchgeführt wird.

2. Verfahren nach Anspruch 1, wobei als Salz des Calcitoninderivats ein Salz einer Mineralsäure, einer organischen Säure, eines Metalls, von Ammoniak oder einer organischen Base hergestellt wird.

3. Verfahren nach Anspruch 2, wobei als Salz des Calcitoninderivats ein Salz der Salzsäure, Schwefelsäure, Phosphorsäure, Essigsäure, Zitronensäure, Natrium, Kalium, Calcium, Ammoniak, Dicyclohexylamin oder Pyridin hergestellt wird.

4. Verfahren zum Herstellen einer pharmazeutischen Zusammensetzung durch Formulieren eines Calcitoninderivats gemäss einem der Ansprüche 1 bis 3 oder eines Salzes davon mit einem pharmakologisch geeigneten Träger.

5. Verwendung eines Calcitoninderivats und dessen Salzes, wobei das Calcitoninderivat durch die Formel

```
                              Acm
                               |
pGlu-Ala-Thr-Leu-Ser-Thr-Cys-Val-Leu-Gly-Lys-Leu-Ser-Gln-
 Glu-Leu-His-Lys-Leu-Gln-Thr-Tyr-Pro-Arg-Thr-Asp-Val-Gly-
 Ala-Gly-Thr-Pro-NH2
```

dargestellt ist, wobei Ala: Alanin, Thr: Threonin, Leu: Leucin, Ser: Serin, Cys: Cystein, Val: Valin, Gly: Glycin, Lys: Lysin, Gln: Glutamin, His: Histidin, Tyr: Tyrosin, Pro: Prolin, Arg: Arginin, Asp: Asparaginsäure, pGlu: Pyroglutaminsäure, Acm: Acetamidomethyl und Glu: Glutaminsäure bedeutet, zum Herstellen eines Arzneimittels, welches wirksam in bezug auf die Verringerung des Serum-$Ca^{2+}$-Spiegels oder wirksam im Hinblick auf die Behandlung von Osteoporose, Paget's Knochenkrankheit oder digestiver Hyperkalzämie ist.

6. Verwendung nach Anspruch 5, wobei das Salz des Calcitoninderivats ein Salz einer Mineralsäure, einer organischen Säure, eines Metalls, von Ammoniak oder einer organischen Base ist.

7. Verwendung nach Anspruch 6, wobei das Salz des Calcitoninderivats ein Salz von Salzsäure, Schwefelsäure, Phosphorsäure, Essigsäure, Zitronensäure, Natrium, Kalium, Calcium, Ammoniak, Dicyclohexylamin oder Pyridin ist.

**8.** Verwendung eines Calcitoninderivats oder dessen Salzes nach einem der Ansprüche 1 bis 3 zum Herstellen eines Arzneimittels.

**Revendications**

**Revendications pour les Etats contractants suivants : CH, DE, FR, GB, IT, LI, SE**

**1.** Dérivé de la calcitonine et son sel correspondant dans lesquels le dérivé de la calcitonine est représenté par la formule:

Acm
|
pGlu-Ala-Thr-Leu-Ser--Thr-Cys-Val-Leu-Gly-Lys-Leu-Ser-Gln-
Glu-Leu-His-Lys-Leu-Gln-Thr-Tyr-Pro-Arg-Thr-Asp-Val-Gly-
Ala-Gly-Thr-Pro-NH2

dans laquelle Ala représente alanine, Thr: thréonine, Leu: leucine, Ser: sérine, Cys: cystéine, Val: valine, Gly: glycine, Lys: lysine, Gln: glutamine: His: histidine, Tyr: tyrosine, Pro: proline, Arg: arginine, Asp: acide aspartique, pGlu: acide pyroglutamique, Acm: acétamidométhyle et Glu: acide glutamique.

**2.** Dérivé de la calcitonine et son sel correspondant selon la revendication 1, dans lesquels un dérivé de la calcitonine salifié est le sel conjugué d'un acide minéral, d'un acide organique, d'un métal, de l'ammoniac ou d'une base organique.

**3.** Dérivé de la calcitonine et son sel correspondant selon la revendication 2, dans lesquels un dérivé de la calcitonine salifié est le sel conjugué de l'acide chlorhydrique, sulfurique, phosphorique, acétique, citrique, du sodium, potassium, calcium, de l'ammoniac, de la cyclohexylamine ou de la pyridine.

**4.** Procédé de préparation d'un dérivé de la calcitonine tel que revendiqué dans l'une quelconque des revendications précédentes par voie de synthèse peptidique sur phase solide ou liquide.

**5.** Composition pharmaceutique comprenant un dérivé de la calcitonine selon l'une quelconque des revendications 1 à 3 ou d'un sel de celui-ci avec un véhicule pharmaceutiquement acceptable.

**6.** Dérivé de la calcitonine selon l'une quelconque des revendications 1 à 3 destiné à être employé comme médicament.

**7.** Dérivé de la calcitonine selon l'une quelconque des revendications 1 à 3 destiné à abaisser le taux de $Ca^{++}$ sérique chez l'animal ou l'homme.

**8.** Utilisation d'un dérivé de la calcitonine selon l'une quelconque des revendications 1 à 3 en vue de préparer un médicament efficace pour abaisser le taux de $Ca^{++}$ sérique ou pour traiter l'ostéoporose, la maladie osseuse de Paget ou l'hypercalcémie digestive.

**Revendications pour les l'Etat contractant suivant : ES**

**1.** Procédé de préparation d'un dérivé de la calcitonine représenté par la formule :

Acm
|
pGlu-Ala-Thr-Leu-Ser--Thr-Cys-Val-Leu-Gly-Lys-Leu-Ser-Gln-
Glu-Leu-His-Lys-Leu-Gln-Thr-Tyr-Pro-Arg-Thr-Asp-Val-Gly-
Ala-Gly-Thr-Pro-NH2

dans laquelle Ala représente alanine, Thr: thréonine, Leu: leucine, Ser: sérine, Cys: cystéine, Val: valine, Gly: glycine, Lys: lysine, Gln: glutamine: His: histidine, Tyr: tyrosine, Pro: proline, Arg: arginine, Asp: acide aspartique, pGlu: acide pyroglutamique, Acm: acétamidométhyle et Glu: acide glutamique, ou d'un sel de celui-ci par voie de synthèse peptidique sur phase solide ou liquide.

**2.** Procédé selon la revendication 1 dans lequel le dérivé de la calcitonine salifié préparé est le sel conjugué d'un acide minéral, organique, d'un métal, de l'ammoniac ou d'une base organique.

**3.** Procédé selon la revendication 2 dans lequel le dérivé de la calcitonine salifié préparé est un sel conjugué de l'acide chlorhydrique, sulfurique, phosphorique, acétique, citrique, du sodium, potassium, calcium, de l'ammoniac, de la dicyclohexylamine ou de la pyridine.

**4.** Procédé de préparation d'une composition pharmaceutique par formulation d'un dérivé de la calcitonine selon l'une quelconque des revendications 1 à 3 ou d'un sel de celui-ci avec un véhicule pharmaceutiquement acceptable.

**5.** Utilisation d'un dérivé de la calcitonine ou d'un sel de celui-ci, dans lequel le dérivé de la calcitonine est représenté par la formule:

Acm
|
pGlu-Ala-Thr-Leu-Ser--Thr-Cys-Val-Leu-Gly-Lys-Leu-Ser-Gln-
Glu-Leu-His-Lys-Leu-Gln-Thr-Tyr-Pro-Arg-Thr-Asp-Val-Gly-
Ala-Gly-Thr-Pro-NH2

dans laquelle Ala représente alanine, Thr: thréonine, Leu: leucine, Ser: sérine, Cys: cystéine, Val: valine, Gly: glycine, Lys: lysine, Gln: glutamine: His: histidine, Tyr: tyrosine, Pro: proline, Arg: arginine, Asp: acide aspartique, pGlu: acide pyroglutamique, Acm: acétamidométhyle et Glu: acide glutamique en vue de préparer un médicament efficace pour abaisser le taux de $Ca^{++}$ sérique ou pour traiter l'ostéoporose, la maladie osseuse de Paget ou l'hypercalcémie digestive.

**6.** Utilisation selon revendication 5 dans laquelle un dérivé de la calcitonine salifié est le sel conjugué d'un acide minéral, organique, d'un métal, de l'ammoniac, ou d'une base organique.

**7.** Utilisation selon la revendication 6 dans laquelle un dérivé de la calcitonine salifié est le sel conjugué de l'acide chlorhydrique, sulfurique, phosphorique, acétique, citrique, du sodium, potassium, calcium, de l'ammoniac, de la dicyclohexylamine ou de la pyridine.

**8.** Utilisation d'un dérivé de la calcitonine ou de son sel correspondant tels que définis dans les revendications 1 à 3 en vue de préparer un médicament.